Europäisches Patentamt

European Patent Office

Office européen des brevets

(19)

(11) Publication number: **0 432 983 A2**

(12) **EUROPEAN PATENT APPLICATION**

(21) Application number: **90313371.8**

(22) Date of filing: **10.12.90**

(51) Int. Cl.5: **C07D 317/70**, C07D 407/10, A61K 31/36

(30) Priority: **15.12.89 GB 8928418**
**28.11.90 GB 9025868**

(43) Date of publication of application:
**19.06.91 Bulletin 91/25**

(84) Designated Contracting States:
**AT BE CH DE DK ES FR GB GR IT LI LU NL SE**

(71) Applicant: **PHARMA MAR, S.A.**
**Paseo de la Castellana, 52 - 7**
**E-28046 Madrid(ES)**

(72) Inventor: **Higa, Tatsuo**
**1-62-3 Shiru Ishimine,**
**7-202 Naha, Okinawa 903(JP)**
Inventor: **Tanaka, Junichi**
**1386 Maeda,**
**18-307 Urasoe, Okinawa 901-21(JP)**
Inventor: **Gravalos, Dolores Garcia**
**Maldonado 63**
**E-28006 Madrid(ES)**

(74) Representative: **Lamb, John Baxter et al**
**MARKS & CLERK 57/60 Lincoln's Inn Fields**
**London WC2A 3LS(GB)**

(54) **Novel compounds of the diterpene type.**

(57) The present invention relates to novel diterpene-type compounds, the production thereof, pharmaceutical compositions comprising these compounds and their use as anti-viral, anti-tumour and cytotoxic agents. The compounds, termed helioporins, may be isolated from coral species, for example the blue coral, Heliopora coerulea.

EP 0 432 983 A2

## NOVEL COMPOUNDS OF THE DITERPENE TYPE

The present invention relates to novel diterpene-type compounds, the production thereof, pharmaceutical compositions comprising these compounds and their use as anti-viral, anti-tumour and cytotoxic agents.

The compounds of the invention belong to the diterpene class and may be produced, for example, by isolation from coral species, such as the blue coral, Heliopora coerulea. They are structurally related to pseudopterosins, seco-pseudopterosins and other similar compounds, all of which may be isolated from Caribbean sea whips [c. f. Look et al, J. Organic Chem 51 (1986) 5140; Look et al, PNAS 83 (1986) 6283; Look et al, Tetrahedron Letters 43 (1987) 3363 and Harvis et al, Tetrahedron Letters 29 (1988) 4361]. Pseudopterosins have been found to possess anti-inflammatory and analgesic activity.

In a first embodiment the present invention provides compounds of formula I

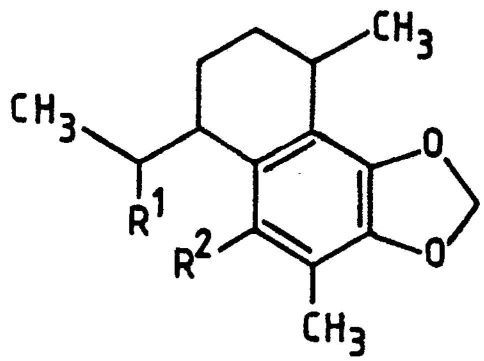

I

wherein $R^1$ is a group of formula II

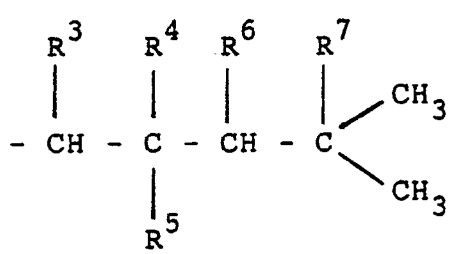

II

in which:

$R^3$ is hydrogen, and

$R^4$ and $R^5$ are each hydrogen, or

$R^4$ and $R^5$ together represent a carbonyl oxygen atom, or

$R^4$ together with $R^2$ represents a carbon-carbon bond and $R^5$ is hydrogen; or

$R^3$ and $R^4$ together represent a carbon-carbon bond and $R^5$ is hydrogen;

$R^6$ and $R^7$ each represent hydrogen, or

$R^6$ and $R^7$ together represent a carbon-carbon bond, or

$R^6$ and $R^7$ together represent an oxirane oxygen atom;

and $R^2$ is hydrogen or, together with $R^4$ forms a carbon-carbon bond.

A preferred group of compounds of the present invention are those in which:

1. $R^3$ is hydrogen, $R^4$ together with $R^2$ represents a carbon-carbon bond, $R^5$ is hydrogen and $R^6$ and $R^7$ together represent an oxirane oxygen atom;

2. $R^2$ and $R^3$ are each hydrogen, $R^4$ and $R^5$ together represent a carbonyl oxygen atom and $R^6$ and $R^7$ each represent hydrogen;

3. $R^2$ and $R^3$ are each hydrogen, $R^4$ and $R^5$ together represent a carbonyl oxygen atom and $R^6$ and $R^7$ together represent a carbon-carbon bond;

4. $R^2$, $R^3$, $R^4$ and $R^5$ are each hydrogen and $R^6$ and $R^7$ together represent a carbon-carbon bond;

5. $R^3$ is hydrogen, $R^4$ together with $R^2$ represents a carbon-carbon bond, $R^5$ is hydrogen and $R^6$ and $R^7$ together represent a carbon-carbon bond;

6. $R^3$ and $R^4$ together represent a carbon-carbon bond, $R^2$ and $R^5$ are each hydrogen and $R^6$ and $R^7$ together represent a carbon-carbon bond.

In the compounds of group 6 above, the two resulting double bonds in the side chain $R^1$ may be trans or cis.

The present invention further provides a method for the production of a compound of formula I as hereinabove defined, in particular a compound of any one of groups 1 to 6 as hereinabove defined, which process comprises isolation of the compound from a coral species, for example the blue coral, Heliopora coerulea.

The method of the invention may be performed by a route conventional for the isolation of such compounds from their natural sources, for example by extraction and chromatography. Extraction is suitably performed in the presence of an alcohol, for example methanol and chromatography is, for example, flash chromatography carried out using hexane/hexane ethyl acetate as eluant.

The method of the invention may be better illustrated with reference to the following example.

## Example

The blue coral Helipora coerulea (20 kg), collected in Okinawa, Japan, is extracted by soaking in methanol (16 l) for several days. After concentration the resulting aqueous suspension is extracted with ethyl acetate to give 14 g of an oil. The oil is first separated by flash chromatography on silica gel [hexane: hexane ethyl acetate (EtOAc) - 1: 1, EtOAc: MeOH - 1: 1] into five fractions. The residue (9.7 9) of fraction 2 eluted with 1: 1 hexane-EtOAc was separated further by flash chromatography into five fractions using the hexane: EtOAc system. The residue (2.3 g) of fractions eluted with 10: 1 and 5: 1 hexane-EtOAc was separated on a Lobar Si-60 column (5: 1 hexane-EtOAc) into three fractions. The second fraction (1.38 g) was subjected to separation by HPLC (RP-18, MeOH) to give the compound of group 1 above, hereinafter referred to as Helioporin A or AH-1, (400 mg) and a compound of group 2 above, hereinafter referred to as Helioporin B or AH-2, (250 mg), and by HPLC (RP-8, MeOH) to give compounds of groups 3 to 5, respectively referred to hereinafter as Helioporin C, or AH-3, (29 mg), Helioporin D, or AN-4, (29 mg) and Helioporin E, or AH-5, (31 mg). The compounds have the following characteristics:

HELIOPORIN A : colourless oil $[\alpha]_D^{24}$ +65° (c = 4.4, $CHCl_3$)

IR ($CCl_4$): 2960, 2930, 2870, 21450, 1420, 1380, 1140, 1120 and 1085 $cm^{-1}$

UV (MeOH): $\lambda_{max}$ 213 (ξ 20,000) and 290 nm (ξ 1900)

$^1H$ NMR ($CDCl_3$): δ 5.92 (1h, d, J=1. 5Hz), 5.86 (1H, d, J=1. 5Hz), 3.08 (1H, ddd, J=4.9, 9.2, 9.8Hz), 2.91 (1H, m), 2.64 (1H, d, J=9.2Hz), 2.24 (3H, s), 2.18 (1H, m), 2.08 (4H, complex), 1.44 (3H, s), 1.33 (3H, s), 1.31 (3H, d, J=7.0Hz), 1.26 (1H, m), 1.16 (1H, m), 1.10 (1H, m), 1. 04 (3H, d, J=6. 1Hz)

$^{13}C$ NMR ($CDCl_3$): δ 144.3 (s), 143. 4 (s), 132.9 (s), 130.0 (s), 121.2 (s), 116.4 (s), 99.9 (t), 70.9 (d), 58.0 (s), 42.9 (d), 35.0 (d), 34.9 (t), 34.8 (d), 32. 2 (t), 30.4 (d), 28.2 (t), 25.0 (q), 21.3 (q), 19.7 (q), 19.2 (q), 12.3 (q)

EIMS m/z: 328 (M+, 20), 257 (96), 256 (100), 241 (53), 228 (38), 227 (12), 213 (14), 199 (34 rel %)

HELIOPORIN B: colourless crystals $[\alpha]_D^{24}$ +18.8° (c=2.55, $CHCl_3$)

IR ($CCl_4$): 2970, 2930, 2880, 1710, 1440, 1415, 1120, 1115, 1070, 1030, 960, 905 $cm^{-1}$

UV (MeOH): $\lambda_{max}$ 212 (ξ 19,500) & 287 nm (ξ 1600)

$^1H$ NMR ($CDCl_3$): δ 6. 54 (1H, s), 5. 93 (1H, d, J=1. 5Hz), 5.87 (1H, d, J=1. 5Hz), 2.93 (1H, sextet, J=6.4Hz), 2.55 (2H, complex), 2. 48 (1H, dd, J=4.9, 16.2 Hz), 2.32 (1H, dd, J=7.9, 16.2Hz), 2.25 (1H, d, J=2.4Hz), 2.24 (1H, d, J=1.5Hz), 2.19 (3H,s), 2.12 (1H, m), 2.01 (1H, m), 1.78 (1H, m), 1. 60 (1H, m), 1.39 (1H, m), 1.23 (3H, d, J=7.0Hz), 0. 91 (3H, d, J=6.7Hz), 0. 90 (3H, d, J= 6.7Hz), 0. 76 (3H, d, J=6.4Hz)

$^{13}C$ NMR ($CDCl_3$): δ 209. 9 (s), 144. 5 (s), 143.2 (s), 132.4 (s), 122.7 (d), 122.6 (s), 115.7 (s), 100.0 (t), 52. 3 (t), 48.8 (t), 41.1 (d), 33.2 (d), 28.5 (t), 28.0 (d),. 24.3 (q), 22.43 (q), 22.38 (t), 20.9 (t), 20.8 (q), 16.1 (q), 14.4 (q)

HELIOPORIN C: colourless oil $[\alpha]_D^{22}$ +8.9° (c=0.58, $CHCl_3$)

$^1H$ NMR ($CDCl_3$): δ 6. .52 (1H, s), 6.03 (1H, s), 5. 90 (1H, s), 5.84 (1H, s), 2.91 (1H, sextet, J=6.3Hz), 2.15 (3H, s), 2.10 (3H, s), 1. 86 (3H, s), 1.20 (3H, d, J=7.2Hz), 0. 75 (3H, d, J=6.0Hz)

$^{13}C$ NMR ($CDCl_3$): δ 201. 4 (s), 155.2 (s). 145.0 (s), 143.7 (s), 133.3 (s), 124.7 (s), 124.7 (d), 123.5 (s), 116.4 (s), 100.7 (t). 50.5 (t). 41.8 (d), 34.5 (d), 29.0 (t), 28.6 (d), 28.1 (q), 21.5 (q), 21.4 (t), 21.1 (q), 16.7 (q), 15.0 (q)

HELIOPORIN D: colourless oil $[\alpha]_D^{23}$ +6.3° (c=0.36, $CHCL_3$)

$^1H$ NMR ($CDCl_3$): δ 6.49 (1H, s), 5.91 (1H, d, J=1.2Hz), 5. 85 (1H, d, J=1.2Hz), 5.12 (1H, t, J=6Hz), 2.90 (1H, sextet, J=6.6Hz), 2.63 (1H, dd, J=10.8, 5.7Hz), 2.17 (3H, s), 1.69 (3H, s,), 1. 60 (3H, s), 1. 22

(3H, d, J=6.9Hz0, 0.68 (3H, d, J=6.9Hz)

$^{13}C$ NMR ($CDCl_3$): δ 145.0 (s), 143.4 (s), 134.2 (s), 131.6 (s), 125.3 (d), 123.5 (s), 122.8 (d), 116.3 (s), 100.6 (t), 41.1 (d), 37.7 (d), 35.9 (t), 30.0 (t), 29.0 (d), 26.7 (t), 26.2 (q), 21.5 (q), 20.9 (t), 18.1 (q), 16.0 (q), 15.1 (q)

HELIOPORIN E: colourless oil $[\alpha]_D^{23}$ +111° (c=0.55, $CHCl_3$)

$^1H$ NMR ($CDCl_3$): δ 5.88 (1H, d, J=1.5Hz), 5.82 (1H, d, J=1.5Hz), 4.93 (1H, dt, J=9.3, 1.5Hz), 3.69 (1H, dt, J=9, 5.7Hz), 2.95 (1H, m), 2.01 (3H, s), 1.70 (3H, d, J=1.2Hz), 1.65 (3H, d, J=1.2Hz), 1.28 (3H, d, J=6.9Hz), 0.99 (3H, d, J=5.7Hz)

$^{13}C$ NMR ($CDCl_3$): δ 144.6 (s), 143.4 (s), 132.6 (s), 132.0 (s), 131.5 (d), 129.0 (s), 121.4 (s), 116.4 (s), 100.3 (t), 45.2 (d), 40.6 (t), 37.0 (d), 34.8 (d), 32.8 (t), 30.3 (d), 28.6 (t), 25.8 (q), 22.3 (q), 20.1 (q), 18.0 (q), 12.3 (q)

HELIOPORIN F: colourless oil, $[\alpha]_D^{23}$ -17° (c=0.55, $CHCl_3$)

$^1H$ NMR ($CDCl_3$): δ 6.53 (1H, s), 6. 10 (1H, dd, J=11.3, 10.7hZ), 5.94 (1H, s), 5.90 (1H, d, J=11.3Hz), 5.87 (1H, s), 5. 29(1H, dd, J=10.7, 10.3 Hz), 3.03 (1H, ddq, J=6.8, 6.8, 6.8Hz), 2.97 (1H, ddq, J=5.4, 5.4, 5.4Hz), 2.18 (3H, s), 2.05 (1H, m), 1.87 (1H, m), 1.77 (3H, s), 1.73 (3H, s), 1.72 (1H, m), 1.40 (1H, m), 1.24 (3H, d, J=7.3Hz), 0.91 (3H, d, J=6.8Hz)

$^{13}C$ NMR ($CDCl_3$): δ 143.3 (s), 135.2 (s), 134.6 (d), 132.7 (s), 123.7 (d), 123.4 (d), 122.8 (s), 122.2 (s), 120.5 (d), 115.8 (s), 100.3 (t), 42.4 (d), 36.6 (d), 28.2 (t), 28.1 (d), 26.4 (q), 21.8 (t), 21.3 (q), 18.1 (q), 17.7 (q), 14.7 (q)

IR ($CCl_4$): 2930, 1420, 1080, 965 $cm^{-1}$

HELIOPORIN G: colourless oil $[\alpha]_D^{22}$ -50° (c=0.69, $CHCl_3$)

$^1H$ NMR ($CDCl_3$): δ 6. 52 (1H, s), 6.17 (1H, dd, J=15.2, 10.7Hz), 5.94 (1H, s), 5.89 (1H, s), 5.83 (1H, d, J=10.7Hz), 5. 61 (1H, dd, J=15.2, 6.4 Hz), 2.96 (1H, ddq, J=6.2, 6.2, 6.8 Hz), 2.69 (2H, m), 2.19 (3H, s), 1.98 (1H, m), 1.80 (1H, m), 1. 78 (3H, s), 1.74 (3H, s), 1.64 (1H, m), 1.39 (1H, m), 1.24 (3H, d, J=6.8Hz), 0.91 (3H, d, J=6.4 Hz)

$^{13}C$ NMR ($CDCl_3$): δ 144.6 (s), 143.2 (s), 137.2 (d), 133.1 (s), 132.7 (s), 125.7 (d), 123.3 (d), 123.0 (s), 115.8 (s), 100.3 (t), 42.3 (d), 41.6 (d), 28.6 (t), 28.2 (d), 26.0 (q), 21.2 (t), 21.1 (q), 18.3 (q), 15.9 (q), 14.7 (q)

IR ($CCl_4$): 2930, 1420, 1080, 965 $cm^{-1}$

Compounds of the invention are active as anti-viral, anti-tumour and cytotoxic agents as may be demonstrated in the following test methods.

In the test methods below, compounds AH-1 (Helioporin A) and AH-2 (Helioporin B) are diluted in MeOH/Acetone, 1: 1, at 500 µg/ml. and compounds AH-3, AH-4 and AH-5 (Helioporins C, D and E, respectively) are diluted in MeOH/Acetone , 1: 1, at 1000 µg/ml.

1. Inhibition of HSV-1 replication (anti-viral effect)

16mm dia. wells are seeded each with 2 x $10^5$ CV-1 cells in 1ml aliquots of MEM10C. After three days, the cells are infected with HSV-1 at 10 PFU (Plaque Forming Units) per cell. The inoculum is replaced, after adsorption for 1.5 hours, in pairs of wells with 0. 5 ml aliquots of MEM5C containing the test concentrations of each of AH-1 and AH-2. Four hours from infection, the cells from two wells are scraped into the medium and frozen to -80° C. [The average of PFU/ml of these samples forms the base for calculating net virus production]. The remaining samples are collected in pairs of wells 24 hours after infection and are also frozen to -80° C. For determination of PFU/ml, each sample is diluted to $10^{-8}$. The results are shown below in Table 1.

TABLE 1

| Compound | Dilution | PFU/ml | %I |
|---|---|---|---|
| AH-1 | 0 | $9.5 \times 10^7$ | 0 |
| | 0.1 | $9.5 \times 10^7$ | 0 |
| | 0.5 | $9.5 \times 10^7$ | 0 |
| | 1.0 | $7.0 \times 10^7$ | 26 |
| | 5.0 | $4.5 \times 10^7$ | 53 |
| | 10.0 | $1.9 \times 10^7$ | 80 |
| | 50.0 | $8.0 \times 10^3$ | 100 |
| AH-2 | 0 | $9.5 \times 10^7$ | 0 |
| | 0.1 | $9.5 \times 10^7$ | 0 |
| | 0.5 | $7.5 \times 10^7$ | 21 |
| | 1.0 | $5.5 \times 10^7$ | 42 |
| | 5.0 | $3.5 \times 10^7$ | 63 |
| | 10.0 | $0.6 \times 10^7$ | 94 |
| | 50.0 | $7.5 \times 10^5$ | 100 |

The $IC_{50}$ for AH-1 and AH-2 in the above test method is 4 µg/ml for AH-1 and 1.6 µg/ml for AH-2.

2. Inhibition of growth of CV-1 cells (cytotoxic effect)

CV-1 cells are seeded into 16 mm dia. wells at $10^4$ cells per well in 1ml aliquots of MEM10C. The following day, the medium is replaced with 0.5 ml aliquots of MEM10C containing the test concentrations of compounds AH-1 and AH-2, i. e. 0, 0.1, 0.5, 1, 5, 10 and 50 µg/ml. Determinations are all carried out in duplicate. Four days after seeding, the cells are trypsinised and counted. Percentage inhibition (%I) relative to the wells without test compound (i.e. the controls) is calculated and the $IC_{50}$ determined. There is no inhibition of growth at any of the concentrations tested and the measured $IC_{50}$ for both AH-1 and AH-2 is over 50 µg/ml.

3. Cytotoxic activity of AH-1 and AH-2

This test is performed according to standard procedures for determination of cytotoxicity of a compound againt virus infected cells. In the present case, CV-1 (monkey kidney fibroblast cells) are infected with HSV-1 and the compounds of the invention tested against these at varying concentrations. Results are shown in Table 3

TABLE 3

| Compound | Solvent | Conc | 40µl | 20µl | 10µl | 5µl |
|---|---|---|---|---|---|---|
| AH-1 | MeOH/Acet | 500µg/ml | 0++ | 0+ | 0- | 0- |
| AH-2 | MeOH/Acet | 500µg/ml | 0++ | 0+ | 0- | 0- |

The numbers show the toxicity throughout the CV-1 cells sheet.

- (-) indicates no reduction in viral plaque production
- (+) indicates a 50% reduction in viral plaque production
- (+ +) indicates an 80% reduction in viral plaque production.

4.

Compounds AH-1 and AH-2 were further tested against L-1210 cells (mouse lymphoma) and VSV (Vesicular Stomatitis Virus). They showed no activity against these cell and virus types.

5.

With compounds AH-3, AH-4 and AH-5, four general anti-tumour assays and two cytotoxicity assays were performed. Current screening protocols were employed using test substance diluted as above and at varying concentrations. Tests were performed against the tumour cells P-388 (mouse DBA/2 lymphoid neoplasm), A-549 (human lung carcinoma), HT-29 (human colon carcinoma) and MCF-7 (human breast carcinoma) and for the cytotoxicity assays against CV-1 (monkey kidney fibroblast) and BHK (Hamster kidney fibroblast). Results are shown in the following Table 4.

Scoring is performed as follows:

- (-) no activity - the number of cells is equal to the control of growth, 0-40% cell inhibition
- (+) low activity - numbers of cells are reduced by approx 50-60%
- (+ +) good activity - numbers of cells are reduced by approx 70-90%
- (+ + +) very good activity - there is 100% cell inhibition

[(+) is the approximate $IC_{50}$]

TABLE 4

| CELL TYPE | CMPD NO | 20 | 10 | 5 | 2 | 1 | 0.5 | 0.2 | $IC_{50}$ µg/ml approx |
|---|---|---|---|---|---|---|---|---|---|
| | | TEST CONC (µg) | | | | | | | |
| P-388 | AH-3 | +++ | +++ | +++ | ++ | + | - | - | 1 |
| | AH-4 | ++ | ++ | ++ | + | - | - | - | 2 |
| | AH-5 | ++ | ++ | - | - | - | - | - | 5-10 |
| A-549 | AH-3 | +++ | +++ | + | - | - | - | - | 5 |
| | AH-4 | ++ | ++ | - | - | - | - | - | 5-10 |
| | AH-5 | - | - | - | - | - | - | - | >20 |
| HT-29 | AH-3 | +++ | +++ | ++ | + | - | - | - | 2 |
| | AH-4 | +++ | +++ | + | - | - | - | - | 5 |
| | AH-5 | +++ | ++ | + | - | - | - | - | 5 |
| MCF-7 | AH-3 | +++ | ++ | + | - | - | - | - | 5 |
| | AH-4 | ++ | + | - | - | - | - | - | 10 |
| | AH-5 | +++ | + | - | - | - | - | - | 10 |
| CV-1 | AH-3 | +++ | ++ | - | - | - | - | - | 5-10 |
| | AH-4 | ++ | + | - | - | - | - | - | 10 |
| | AH-5 | ++ | - | - | - | - | - | - | 10-20 |
| BHK | AH-3 | +++ | +++ | +++ | +++ | + | - | - | 1 |
| | AH-4 | +++ | +++ | ++ | - | - | - | - | 2-5 |
| | AH-5 | +++ | +++ | + | - | - | - | - | 5 |

6.

With compounds AH-3, AH-4 and AH-5, two general anti-viral assays were performed. Current screening protocols were employed using test substance diluted as above and at varying concentrations. Tests were performed against HSV-1 (herpes simplex virus, type 1) in CV-1 cells and VSV (vesicular stomatitis virus) in BHK cells. The results are shown below in Table 5.

The activity is measured by the following criteria:

(-) indicates no inhibition of virus replication
(+) indicates that plaques are reduced by approximately 50-60%
(++) indicates that plaques are reduced by approximately 70-90%
(+++) indicates that there is 100% plaque reduction
[(+) is the approximate $IC_{50}$]

Numbers in the table indicate the cytotoxiciyt of the cells, which is determined by the inhibition zone on the cell sheet.

TABLE 5

| CELL TYPE | CMPD NO | TEST CONC (µg) 20 | 10 | 5 | 1 | 0.5 | $IC_{50}$ µg/ml approx |
|---|---|---|---|---|---|---|---|
| CV-1/ | AH-3 | 10+ | 0- | 0- | 0- | 0- | 40 |
| HSV-1 | AH-4 | 0- | 0- | 0- | 0- | 0- | >40 |
| | AH-5 | 0- | 0- | 0- | 0- | 0- | >40 |
| BHK/ | AH-3 | 10- | 8- | 6- | 0- | 0- | >40 |
| VSV | AH-4 | 0- | 0- | 0- | 0- | 0- | >40 |
| | AH-5 | 0- | 0- | 0- | 0- | 0- | >40 |

From the above test methods it may be seen that compounds of formula I, in particular compounds of groups 1 to 6 as hereinbefore defined, are indicated for use as anti-viral, anti-tumour and cytotoxic agents.

Thus, in a further series of embodiments, the present invention provides:

A. the use of a compound of formula I an anti-viral, a cytotoxic and/or an anti-tumour agent, in particular,

(a) the use of a compound of formula I as an anti-viral agent,

(b) the use of a compound of formula I as a cytotoxic agent,

(c) the use of a compound of formula I as an anti-tumour agent.

In a yet further series of embodiments, the present invention provides:

B. a pharmaceutical composition comprising a compound of formula I together with a pharmaceutically acceptable carrier or diluent therefor, preferably:

(d) a pharmaceutical composition comprising a compound of any one of groups 1 to 6, as hereinbefore defined, together with a pharmaceutically acceptable diluent or carrier therefor.

## Claims

**1.** Compounds of formula I

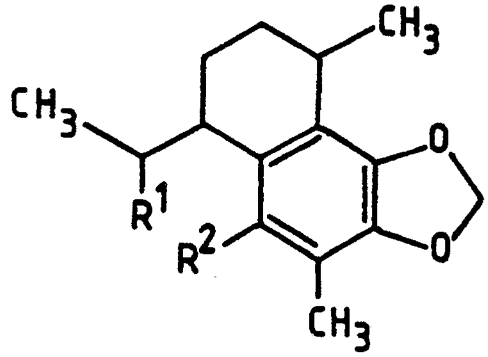

I

wherein $R^1$ is a group of formula II

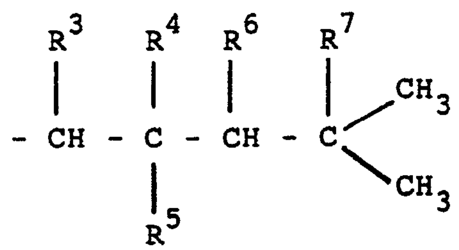

II

in which:

$R^3$ is hydrogen, and

$R^4$ and $R^5$ are each hydrogen, or

$R^4$ and $R^5$ together represent a carbonyl oxygen atom, or

$R^4$ together with $R^2$ represents a carbon-carbon bond and $R^5$ is hydrogen; or

$R^3$ and $R^4$ together represent a carbon-carbon bond and $R^5$ is hydrogen;

$R^6$ and $R^7$ each represent hydrogen, or

$R^6$ and $R^7$ together represent a carbon-carbon bond, or

$R^6$ and $R^7$ together represent an oxirane oxygen atom;

and $R^2$ is hydrogen or, together with $R^4$ forms a carbon-carbon bond.

2. A compound according to claim 1, wherein
   (a) $R^3$ is hydrogen, $R^4$ together with $R^2$ represents a carbon-carbon bond, $R^5$ is hydrogen and $R^6$ and $R^7$ together represent an oxirane oxygen atom; or
   (b) $R^2$ and $R^3$ are each hydrogen, $R^4$ and $R^5$ together represent a carbonyl oxygen atom and $R^6$ and $R^7$ each represent hydrogen; or
   (c) $R^2$ and $R^3$ are each hydrogen, $R^4$ and $R^5$ together represent a carbonyl oxygen atom and $R^6$ and $R^7$ together represent a carbon-carbon bond; or
   (d) $R^2$, $R^3$, $R^4$ and $R^5$ are each hydrogen and $R^6$ and $R^7$ together represent a carbon-carbon bond; or
   (e) $R^3$ is hydrogen, $R^4$ together with $R^2$ represents a carbon-carbon bond, $R^5$ is hydrogen and $R^6$ and $R^7$ together represent a carbon-carbon bond; or
   (f) $R^3$ and $R^4$ together represent a carbon-carbon bond, $R^2$ and $R^5$ are each hydrogen and $R^6$ and $R^7$ together represent a carbon-carbon bond.

3. Method for the production of compounds of formula I as defined in claim 1 which comprises isolation from a coral species using alcohol extraction.

4. Method according to claim 3 wherein the coral species is the blue coral, Heliopora coerulea.

5. Use of a compound of formula I as defined in claim 1 as an anti-viral, anti-tumour or cytotoxic agent.

6. Pharmaceutical composition comprising a compound of formula I as defined in claim 1 together with a pharmaceutically acceptable carrier or diluent therefor.